# EUROPEAN PATENT APPLICATION

(11) **EP 2 987 869 A1**
(43) Date of publication of application: **24.02.2016**
(21) Application number: 14784980.6
(22) Date of filing: 15.04.2014
(51) Int. Cl.: C12Q 1/68, C12N 15/11

(54) **METHOD FOR DIAGNOSING MYOTONIC DYSTROPHY TYPE 1**

(30) Priority: 18.04.2013 KR 20130042873
(71) Applicant: Samsung Life Public Welfare Foundation, Seoul 140-893 (KR); Green Cross Genome Corporation, Yongin-si, Gyeonggi-do 446-770 (KR)
(72) Inventor: KI, Chang Seok, Gangnam-gu, Seoul 135-710 (KR)
(74) Representative: Krauss, Jan
(86) International application number: PCT/KR2014/003238
(87) International publication number: WO 2014/171698

(57) **Abstract**

The present invention relates to a method for diagnosing myotonic dystrophy type 1 or a method for identifying myotonic dystrophy type 1 patients by using a computer processor. The method of the present invention is an early diagnosis method of a genetic disorder having no effective prevention method except prevention in the early months of pregnancy, and can be applied to a method for diagnosing various dominant or recessive genetic disorders in which a specific repeating base sequence of a specific gene is abnormal. According to the method of the present invention, it is possible to take suitable measures related to symptoms, which will occur later, by classifying a genetic carrier and first to third risk groups according to the repetition number of the CTF sequence of 3'-noncoding region of the DDMPK gene. Particularly, the method of the present invention numerically provides a genetic carrier or the approximate prevalence of a disease with respect to an unborn baby, thereby allowing the risk of disorders to be accurately understood.

<110>	SAMSUNG LIFE PUBLIC WELFARE FOUNDATION

<120>	A Method for Diagnosing Myotonic Dystrophy Type 1

<130>	PN130041

<160>	8

<170>	KopatentIn 2.0

<210>	1
<211>	22
<212>	DNA
<213>	Artificial Sequence

<220>
<223>	DM1-F

<400>	1
gaagggtcct tgtagccggg aa	22

<210>	2
<211>	20
<212>	DNA
<213>	Artificial Sequence

<220>
<223>	DM1-R

<400>	2
ggaggatgga acacggacgg	20

<210>	3
<211>	24
<212>	DNA
<213>	Artificial Sequence

<220>
<223>	Amelogenin-GS-F

<400>	3
ccctgggctc tgtaaagaat agtg	24

<210>	4
<211>	24
<212>	DNA
<213>	Artificial Sequence

<220>
<223>	Amelogenin-GS-R

<400>	4
atcagagctt aaactgggaa gctg	24

<210>	5
<211>	1378
<212>	DNA
<213>	Artificial Sequence

<220>

<223>    DMPK-probe

<400>    5
   ggatccacct tcccatgtaa gacccctctc tttcccctgc ctcagacctg ctgcccattc        60
   tgcagatccc ctccctggct cctggtctcc ccgtccagat atagggctca ccctacgtct        120
   ttgcgacttt agagggcaga agccctttat tcagccccag atctccctcc gttcaggcct        180
   caccagattc cctccgggat ctccctagat aacctcccca acctcgattc cgctcgctgt        240
   ctctcgcccc accgctgagg gctgggctgg gctccgatcg ggtcacctgt cccttctctc        300
   tccagctaga tggccccccg gccgtggctg tgggccagtg cccgctggtg gggccaggcc        360
   ccatgcaccg ccgccacctg ctgctccctg ccagggtacg tccggctgcc cacgcccccc        420
   tccgccgtcg cgccccgcgc tccacccgcc ccgtgccacc cgcttagctg cgcatttgcg        480
   gggctgggcc cacggcagga gggcggatct tcgggcagcc aatcaacaca ggccgctagg        540
   aagcagccaa tgacgagttc ggacgggatt cgaggcgtgc gagtggacta acaacagctg        600
   taggctgttg gggcgggggc ggggcgcagg gaagagtgcg ggcccaccta tgggcgtagg        660
   cggggcgagt cccaggagcc aatcagaggc ccatgccggg tgttgacctc gccctctccc        720
   cgcaggtccc taggcctggc ctatcggagg cgctttccct gctcctgttc gccgttgttc        780
   tgtctcgtgc cgccgccctg ggctgcattg ggttggtggc ccacgccggc caactcaccg        840
   cagtctggcg ccgcccagga gccgcccgcg ctccctgaac cctagaactg tcttcgactc        900
   cggggccccg ttggaagact gagtgcccgg ggcacggcac agaagccgcg cccaccgcct        960
   gccagttcac aaccgctccg agcgtgggtc tccgcccagc tccagtcctg tgatccgggc        1020
   ccgcccccta gcggccgggg agggaggggc cgggtccgcg gccggcgaac ggggctcgaa        1080
   gggtccttgt agccgggaat gctgctgctg ctgctgctgc tgctgctgct gctgggggga        1140
   tcacagacca tttctttctt tcggccaggc tgaggccctg acgtggatgg gcaaactgca        1200
   ggcctgggaa ggcagcaagc cgggccgtcc gtgttccatc ctccacgcac ccccacctat    1260
   cgttggttcg caaagtgcaa agctttcttg tgcatgacgc cctgctctgg ggagcgtctg        1320
   gcgcgatctc tgcctgctta ctcgggaaat ttgcttttgc caaacccgct ttttcggg        1378

<210>    6
<211>    20
<212>    DNA
<213>    Artificial Sequence

<220>
<223>    DMPK-P1-F

<400>    6
ggggctcgaa gggtccttgt                                        20

<210>    7
<211>    42
<212>    DNA
<213>    Artificial Sequence

<220>
<223>   DMPK-P4CAG-R

<400>   7
agcggataac aatttcacac aggacagcag cagcagcagc ag                    42

<210>   8
<211>   24
<212>   DNA
<213>   Artificial Sequence

<220>
<223>   DMPK-P3-R

<400>   8
agcggataac aatttcacac agga                                        24

## Description

### Technical Field

This application claims priority to and the benefit of Korean Patent Application No. 10-2013-0042873 filed in the Korean Intellectual Property Office on 18 April 2013, the entire contents of which are incorporated herein by reference.

The present invention relates to a method for diagnosing myotonic dystrophy type 1.

### Background Art

Myotonic dystrophy type 1 is one of the most common genetic muscular diseases in Koreans, and is an autosomal dominant disease characterized in mytonia and progressive muscular weakness. This disease causes disorders in several organs, such as heart, eyes, endocrine organs, digestive organs, and the central nervous system, together with muscle-relating symptoms (Harper PS et al., DG M. Myotonic dystrophy, in Engel AG, Franzini-Armstrong C (eds): Myology (ed3). In. New York: NY, McGraw Hill Professional, 2004:1039-76). Myotonic dystrophies are classified into myotonic dystrophy type 1 (DM 1) and myotonic dystrophy type 2 (DM 2) according to the causative gene. Myotonic dystrophy type 1 is caused by an abnormal expansion of the CTG trinucleotide sequence in the 3'-untranslated region of the dystrophia myotonica protein kinase (DMPK) gene on chromosome 19, and myotonic dystrophy type 2 is caused by an unstable expansion of the CCTG tetranuecleotide sequence in intron 1 of the zinc finger protein 9 (ZNF9) on chromosome 3q21.

Especially, myotonic dystrophy type 1 results in physical disabilities at adulthood, and thus the diagnosis of myotonic dystrophy type 1 has been performed on patients who already show symptoms or family members of patients diagnosed with the disease. In the conventional art, the diagnosis of the disease has been researched by investigating the degree of variation of the DMPK gene using the molecular method, and PCR or southern blot analysis is mainly used.

Meanwhile, myotonic dystrophy type 1 has no particular symptoms, but its early diagnosis and prevention are important since its symptoms are mainly shown in adults in their 20s and 30s and it is entailed on posterity in an autosomal dominant form. In the conventional art, the diagnosis of myotonic dystrophy type 1 has been performed on patients who already show symptoms or family members of patients diagnosed with the disease. However, it is more important to determine carriers of this disease and the risk of inheritance to posterity.

Therefore, the diagnosis of myotonic dystrophy type 1 is important in women of childbearing age and fetuses, and the diagnosis method of the present invention enables more active preparation by predicting the possibility of incidence of the disease and severity of the disease. Myotonic dystrophy type 1, which is a hereditary disease, has no effective preventive method, excluding the prevention prior to pregnancy or at the early stage of pregnancy. Therefore, the diagnostic method of the disease needs to be secured.

Throughout the entire specification, many papers and patent documents are referenced and their citations are represented. The disclosure of cited papers and patent documents are entirely incorporated by reference into the present specification, and the level of the technical field within which the present invention falls, and details of the present invention are explained more clearly.

### Detailed Description of the Invention

### Technical Problem

The present inventor has researched and endeavored to develop a method for early diagnosis of myotonic dystrophy type 1. As a result, the present inventor has developed a diagnostic method for determining the incidence or not of a disease by, based on the fact that the disease is caused by an expansion of CTG repeats when carriers or patients with mild symptoms, who have alleles with increased CTG repeats in the DMPK gene, give birth to children, finding out carriers or patients with mild symptoms from a target group of pregnant women, women of childbearing age, or newborn babies, through a screening test, and then performing a test on fetuses, and thus has completed the present invention.

Therefore, an aspect of the present invention is to provide a method for diagnosing myotonic dystrophy type 1.

Another aspect of the present invention is to provide a computer-implemented identification method of myotonic dystrophy type 1 patients.

Other purposes and advantages of the present disclosure will become more obvious with the following detailed description of the invention, claims, and drawings.

### Technical Solution

In accordance with an aspect of the present invention, there is provided a diagnostic method of detecting CTG repeats in the dystrophia myotonica-protein kinase (DMPK) gene in order to provide information necessary for the diagnosis of myotonic dystrophy type 1, the method including:
(a) performing a first diagnostic test on a target group composed of pregnant women, women of childbearing age, or newborn babies, to screen positive individuals, the first diagnostic test being performed by measuring the number of CTG repeats in the 3'-untranslated region of the DMPK gene in a nucleic acid isolated from a biological sample of the target group, wherein, (i) an individual is determined as being positive if the individual is a homozygote in which two alleles in the DMPK gene have 34 or less CTG repeats or a heterozygote in which two alleles in the DMPK gene have 34 or less and 35 or more CTG repeats, respectively; (ii) an individual is determined as being negative if the individual is a heterozygote in which two alleles in the DMPK gene have 34 or less CTG repeats; or (iii) an individual is determined as being positive if otherwise; and
(b) performing a second diagnostic test on the positive individuals in step (a) to screen positive individuals, the second diagnostic test being performed by measuring the number of CTG repeats in the 3'-untranslated region of the DMPK gene in a nucleic acid isolated from a biological sample of the positive individuals in step (a), wherein, (i) an individual is determined as being negative, if the individual has been determined as being positive as a homogygote in which two alleles in the DMPK gene have 34 or less CTG repeats in the first diagnostic test but the individual has no allele with 35 or more CTG repeats found between the two alleles in the DMPK gene in the second diagnostic test; and (ii) an individual is determined as being positive, if the individual has been the positive individual in the first diagnostic test and the individual has an allele with 35 or more CTG repeats found beweeen the two alleles in the DMPK gene in the second diagnostic test.

The present inventor has researched and endeavored to develop a method for early diagnosis of myotonic dystrophy type 1. As a result, the present inventor has developed a diagnostic method for determining the incidence or not of a disease by, based on the fact that the disease is caused by an expansion of CTG repeats when carriers or patients with mild symptoms, who have alleles with increased CTG repeats in the DMPK gene, give birth to children, finding out carriers or patients with mild symptoms from a target group of pregnant women, women of childbearing age, or newborn babies, through a screening test, and then performing a test on fetuses.

The diagnostic method of the present invention can be applied to the screening of not only myotonic dystrophy type 1, which is an autosomal dominant disorder, but also other genetic diseases caused by abnormal genes, and thus can provide important information on complications due to the progress of the genetic diseases or acquired disorders.

The diagnostic method of the present invention is set forth as a "diagnostic method of detecting CTG repeats in the dystrophia myotonica-protein kinase (DMPK) gene in order to provide information necessary for the diagnosis of myotonic dystrophy type 1", but may be expressed as a "method of detecting CTG repeats in the dystrophia myotonica-protein kinase (DMPK) gene in order to provide information necessary for the diagnosis of myotonic dystrophy type 1" or a "method for diagnosing myotonic dystrophy type 1, the method including a step of identifying CTG repeats in the dystrophia myotonica-protein kinase (DMPK) gene of an animal biological sample isolated *ex vivo.*

As used herein, the term "biological sample" refers to an animal biopsy sample isolated *ex vivo,* and means a blood, plasma, serum, urine, cell, hair, or tissue sample. According to a specific embodiment of the present invention, the biological sample includes blood, hair, or oral mucosal cells. Meanwhile, the biological sample includes chorion, amniotic fluid, placenta, or cord blood in cases where the subject of diagnosis is a fetus.

As used herein, the term "nucleic acid" refers to comprehensively including DNA (gDNA and cDNA) and RNA molecules, and the nucleotide as a basic constituent unit in the nucleic acid molecule includes naturally occurring nucleotides, and analogues with modified sugars or bases (Scheit, Nucleotide Analogs, John Wiley, New York (1980); Uhlman, and Peyman, Chemical Reviews, 90:543-584(1990)).

The method for diagnosing myotonic dystrophy type 1 according to the present invention will be described in detail as below.

### Step (a): First diagnostic test

First, a first diagnostic test was performed on a target group composed of pregnant women, women of childbearing age, or newborn babies to select positive individuals.

The first diagnostic test is performed by measuring the number of CTG repeats in the 3'-untranslated region of the DMPK gene in a nucleic acid isolated from a biological sample of the target group. That is, whether or not the CTG sequence is repeated or the number of CTG repeats may be measured through an amplification reaction of the 3'-untranslated region of the DMPK gene.

As used herein, the term "amplification reaction" refers to a reaction of amplifying a gene or nucleic acid molecule. Various amplification reactions have been reported in the art, including a polymerase chain reaction (hereinafter, referred to as "PCR") (USP 4,683,195, 4,683,202, and 4,800,159), a reverse transcription-polymerase chain reaction (hereinafter, designated by RT-PCR) (Sambrook et al., Molecular Cloning. A Laboratory Manual, 3rd ed. Cold Spring Harbor Press (2001)), methods of Miller, H. I. (WO 89/06700), and Davey, C. et al. (EP 329,822), a ligase chain reaction (LCR) (17, 18), Gap-LCR (WO 90/01069), a repair chain reaction (EP 439,182), transcription-mediated amplification (TMA) (19) (WO 88/10315), self-sustained sequence replication (20) (WO 90/06995), selective amplification of target polynucleotide sequences (USP 6,410,276), a consensus sequence primed polymerase chain reaction (CP-PCR) (USP 4,437,975), an arbitrarily primed polymerase chain reaction (AP-PCR) (USP 5,413,909 and 5,861,245), nucleic acid sequence based amplification (NASBA) (USP 5,130,238, 5,409,818, 5,554,517, and 6,063,603), and loop-mediated isothermal amplification (LAMP) (23), but are not limited thereto. Other usable amplification methods are described in USP 5,242,794, 5,494,810, and 4,988,617, and US Patent Application Serial no. 09/854,317.

PCR is the most well known method for gene or nucleic acid amplification, and modifications and applications thereof have been developed. For the diagnostic method of the present invention, not only traditional PCR methods but also touchdown PCR (24), hot start PCR (25, 26), nested PCR (2), and booster PCR (27), which are developed by modifying traditional PCR procedures in order to improve PCR specificity or sensitivity, may be employed. In addition, real-time PCR, differential display PCR (DD-PCR), rapid amplification of cDNA ends (RACE), inverse polymerase chain reaction (IPCR), vectorette PCR, thermal asymmetric interlaced PCR (TAIL-PCR), and multiplex PCR may be employed. Detailed descriptions of PCR are shown in McPherson, M.J., and Moller, S.G. PCR. BIOS Scientific Publishers, Springer-Verlag New York Berlin Heidelberg, N.Y. (2000), the teaching of which is incorporated by reference herein.

The diagnostic method of the present invention may be performed using hybridization, immunoassay, or microarray, in addition to the above-mentioned gene amplification methods.

A probe or primer used in the diagnostic method of the present invention has a sequence complementary to the CTG repeats in the 3'-untranslated region of the dystrophia myotonica-protein kinase (DMPK) gene. As used herein, the term "complementary" refers to having such complementarity as to be selectively hybridizable with the 3'-untranslated region of the DMPK gene under specific hybridization or annealing conditions. Therefore, the term "complementary" has a different meaning from being perfectly complementary. The primer or probe of the present invention may have one or more mismatch nucleotide sequences as long as it is selectively hybridizable with the 3'-untranslated region of the DMPK gene.

A label for the probe can provide a signal for allowing the detection of hybridization or not, and may be conjugated to the oligonucleotide. An appropriate label includes fluorophores (e.g., fluorescein, phycoerythrin, rhodamine, lissamine, and Cy3 and Cy5 (Pharmacia)), chromophores, chemiluminophores, magnetic particles, radioisotopes (P32 and S35), mass labels, electron-dense particles, enzymes (alkaline phosphatase or horseradish peroxidase), cofactors, substrates of enzymes, heavy metals (e.g., gold), antibodies, streptavidin, biotin, digoxigenin, and haptens having specific binding partners such as a chelating group, but is not limited thereto. Labeling may be performed through various methods that are typically performed in the art, such as, a nick translation method, a random priming method (Multiprime DNA labeling systems booklet, "Amersham"(1989)), and a kination method (Maxam & Gilbert, Methods in Enzymology, 65:499(1986)). The labels provide signals that can be detected by fluorescence, radioactivity, chromophore measurement, weight measurement, X-ray diffraction or absorption, magnetism, enzymatic activity, mass analysis, binding affinity, hybridization high frequency, and nanocrystals.

In the method of the present invention, the CTG repeats may be conformed through GeneScan Genetic Analysis of products of the above-described polymerase chain reaction. In the GeneScan Genetic Analysis, two observed peaks are determined as a heterozygote with two identified alleles, and one observed peak is determined as a homozygote.

According to the presetn invention, (i) an individual is determined as being positive if the individual is a homozygote in which two alleles in the DMPK gene have 34 or less CTG repeats or a heterozygote in which two alleles in the DMPK gene have 34 or less and 35 or more CTG repeats, respectively; (ii) an individual is determined as being negative if the individual is a heterozygote in which two alleles in the DMPK gene have 34 or less CTG repeats; or (iii) an individual is determined as being positive if otherwise.

### Step (b): Second diagnostic test

Then, a second diagnostic test is performed on the positive individuals in step (a) to screen positive individuals. In the second diagnostic test, a thorough test is performed on the positive individuals in step (a). The second diagnostic test is performed by measuring the number of CTG repeats in the 3'-untranslated region of the DMPK gene in a nucleic acid isolated from a biological sample of the positive individuals in step (a).

According to a specific embodiment of the present invention, the second diagnostic test may employ southern blot analysis, which is DNA-DNA hybridization, or triplet repeat-primed PCR (TR PCR).

Detailed descriptions of triplet repeat-primed PCR are shown in Radvansky et al., Effect of Unexpected Sequence Interruptions to Conventional PCR and Repeat Primed PCR in Myotonic Dystrophy Type 1 Testing, Diagnostic Molecular Pathology (2011); and WO 2011/097503, the teaching of which is incorporated by reference herein.

According to the present invention, (i) an individual is determined as being negative, if the individual has been determined as being positive as a homozygote in which two alleles in the DMPK gene have 34 or less CTG repeats in the first diagnostic test but the individual has no allele with 35 or more CTG repeats found between the two alleles in the DMPK gene in the second diagnostic test; and (ii) an individual is determined as being positive, if the individual has been the positive individual in the first diagnostic test and the individual has an allele with 35 or more CTG repeats found between the two alleles in the DMPK gene in the second diagnostic test.

According to an embodiment of the presetn invention, the diagnostic method of the present invetnion may further incude (c), when the positive individuals in step (b) are pregnant women, performing a third diagnostic test on fetuses to screen positive individuals. The third diagnostic test is performed by measuring the number of CTG repeats in the 3'-untranslated region of the DMPK gene in a nucleic acid isolated from a biological sample of the fetuses, wherein, (i) an individual is determined as being negative if two alleles in the DMPK gene have 34 or less CTG repeats; and (ii) an individual is determined as being positive if an allele with 35 or more CTG repeats is found in the two alleles in the DMPK gene.

According to an embodiment of the presetn inveiton, the diagnostic method of the presetn inveitno may further include determining the positive individual in the third diagnostic test as being a carrier of myotonic dystrophy in cases of 35-49 CTG repeats, a first risk group of myotonic dystrophy with a high likelihood of mild symptoms in cases of 50-99 CTG repeats, a second risk group of myotonic dystrophy with a high likelihood of being a typical myotonic dystrophy type 1 patient in cases of 100-999 CTG repeats, and a third risk group of myotonic dystrophy with a high likelihood of being a myotonic dystrophy type 1 patient in cases of 1000 or more CTG repeats.

The first risk group means a group in which the likelihood of myotonic dystrophy type 1 is 100%, and myotonic dystrophy type 1 may occur in people in their 20s to 70s and produce mild symptoms and complications, such as cataracts; the second risk group means a group in which the likelihood of myotonic dystrophy type 1 is 100%, and myotonic dystrophy type 1 may occur in people in their 10s to 30s and produce typical mytonia and muscular weakness and most complications, such as cataracts and arrhythmia; and the third risk group means a group in which the likelihood of myotonic dystrophy type 1 is 100%, and myotonic dystrophy type 1 may produce muscular hypotonia, difficulty in breathing, or intelligence retardation during the neonatal period, and typical mytonia before 10 years old.

The third diagnostic test on the fetuses includes a procedure of isolating nucleic acid (DNA) from chorion, amniotic fluid, or cord blood, and includes PCR or southern blot analysis for investigating the number of CTG repeats in the 3'-UTR region of the DMPK gene of the fetus from the isolated nucleic acid. In addition, in order to accurately investigate CTG repeats in the fetus, the third diagnostic test may include a procedure for investigating the number of CTG repeats in the 3'-UTR region of the DMPK gene of the pregnant woman and the biological father.

In accordance with another aspect of the present invention, there is provided a computer-implemented identification method for myotonic dystrophy type 1 patients, the method including:
(a) performing first screening on a target group composed of pregnant women, women of childbearing age, or newborn babies, using a computer processor, to screen positive individuals, wherein the first screening is performed by (i) screening, as a positive individual, a homozygous individual in which two alleles in the DMPK gene have 34 or less CTG repeats or a heterozygous individual in which two alleles in the DMPK gene have 34 or less and 35 or more CTG repeats, respectively; (ii) screening, as a negative individual, a heterozygous individual in which two alleles in the DMPK gene have 34 or less CTG repeats; or (iii) screening, as a positive individual, if otherwise; and
(b) performing second screening on the positive individuals in step (a), using a computer processor, to screen positive individuals, the second screening is performed by (i) screening, as a negative individual, an individual who has been determined as being a positive individual as a homozygote in which two alleles in the DMPK gene have 34 or less CTG repeats in the first screening but has no allele with 35 or more CTG repeats found between the two alleles in the DMPK gene in the second screening; and (ii) screening, as a positive individual, an individual who has been the positive individual in the first screening and has an allele with 35 or more CTG repeats found between the two alleles in the DMPK gene in the second screening.

The computer-implemented identification method for myotonic dystrophy type 1 patients of the present invention employs the method of detecting the CTG repeats in the dystrophia myotonica-protein kinase (DMPK) gene, like the forgoing method for diagnosing myotonic dystrophy type 1 of the present invention, and thus the overlapping descriptions therebetween are omitted to avoid excessive complexity of the specification due to repetitive descriptions thereof.

As used herein, the term "computer processor" is an apparatus for processing a plurality of pieces of diagnostic information in association with CTG repeats of the DMPK genes of multiple individuals, implemented by experimenters, and the plurality of pieces of diagnostic information input in the database may be sorted based on a predetermined standard by the experimenters.

The identification method of the present invention may further include, after the second screening, third screening of profiling various information on fetuses who are screened as being negative in the second screening. That is, the identification method of the present invention may further include creating profiles with respect to the likelihood, severity, phenotype, and clinical aspects, distribution, disease progression, and related complications of the genetic disease, for the fetuses who are screened as being positive in the second screening, and creating profiles with respect to the likelihood of genetic disease, a carrier or not of the genetic disease, and the number of CTG repeats, for the fetuses who are screened as being negative in the second screening.

According to an embodiment of the present invention, the identification method of the present invention may further include, screening the positive individual in the third screening as a carrier of myotonic dystrophy in cases of 35-49 CTG repeats, a first risk group of myotonic dystrophy in cases of 50-99 CTG repeats, a second risk group of myotonic dystrophy in cases of 100-999 CTG repeats, and a third risk group of myotonic dystrophy in cases of 1000 or more CTG repeats.

The method of the present invention may further include displaying the foregoing first to third screening results on a screen of a computer processor with a screen display.

### Advantageous Effects

Features and advantages of the present invention are summarized as follows.
(a) The present invention provides a method for diagnosing myotonic dystrophy type 1 or a method for identifying a myotonic dystrophy type 1 patient, using a computer processor.
(b) The method of the present invention is directed to an early diagnostic method for genetic diseases which are not effectively prevented, besides the prevention in early pregnancy, and can be applied to a diagnostic method of various dominant or recessive hereditary diseases caused by abnormality in the particular nucleotide repeats in the particular gene.
(c) According to the method of the present invention, suitable measures associated with future symptoms can be taken by classifying subjects into carriers, and first to third risk groups depending on the number of CTG repeats in the 3'-untranslated region of the DMPK gene.
(d) Especially, the method of the present invention numerically provides carriers or the degree of incidence of the disease for fetuses, thereby accurately grasping the risk of diseases.

### Brief Description of the Drawings

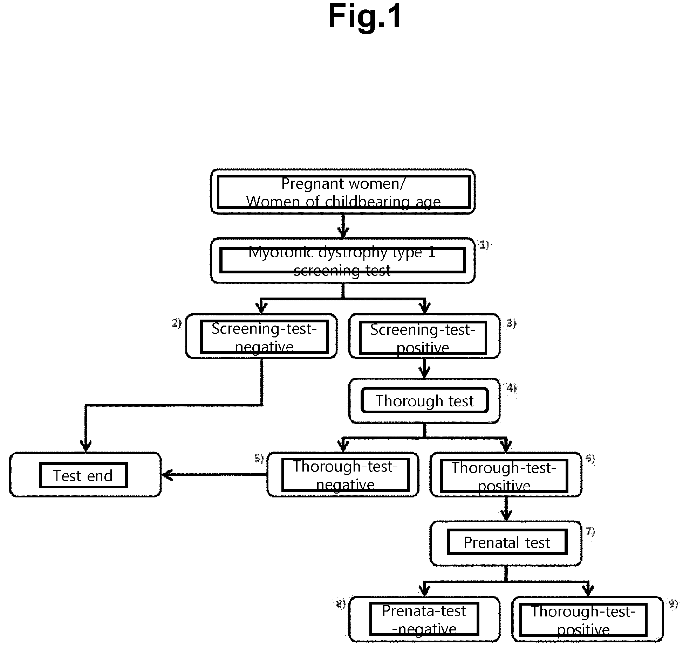

FIG. 1 is a schematic view showing a method for diagnosing myotonic dystrophy type 1 through the diagnostic method of the present invention for pregnant women or women of childbearing age.

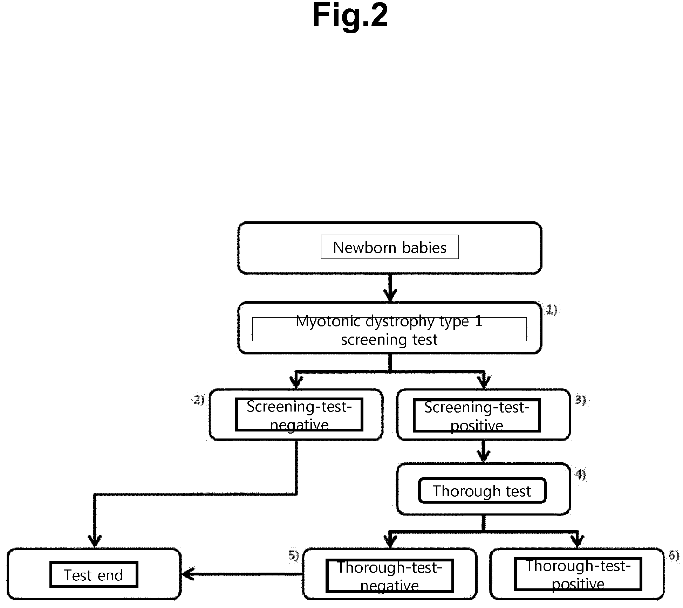

FIG. 2 is a schematic view showing a method for diagnosing myotonic dystrophy type 1 through the diagnostic method of the present invention for newborn babies.

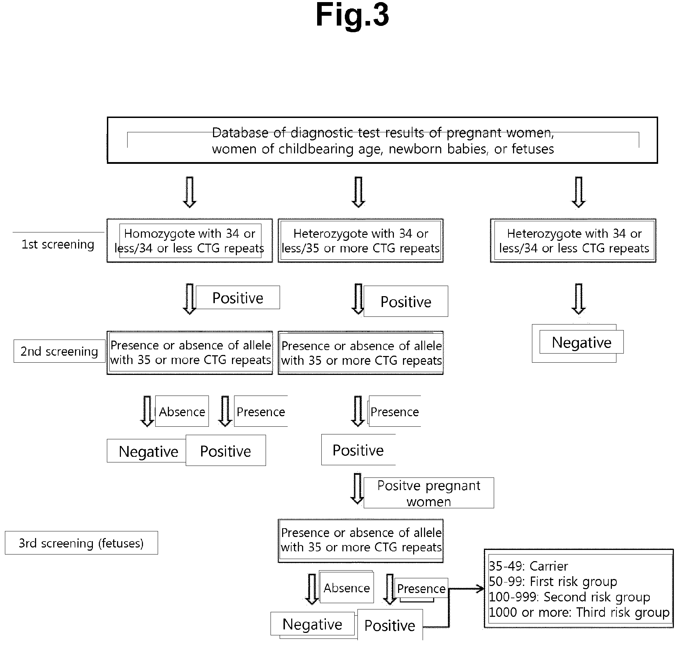

FIG. 3 shows a database flow by a computer-implemented identification method for myotonic dystrophy type 1 patients.

### Mode for Carrying Out the Invention

Hereinafter, the present invention will be described in detail with reference to examples. These examples are only for illustrating the present invention more specifically, and it will be apparent to those skilled in the art that the scope of the present invention is not limited by these examples.

### Examples

### Example 1: Myotonic dystrophy type 1 screening test for pregnant women or women of childbearing age

### DNA sample preparation

Pregnant women or women of childbearing age, who visited Gangnam Severance Hospital, Yonsei University (Seoul, Korea), were diagnosed for myotonic dystrophy type 1, and blood samples were collected from the target group for diagnosis. DNA was isolated from the blood samples following the manufacture's protocol using the "High Pure PCR Template Preparation Kit" (Roche), and the extracted DNA were kept frozen at -20°C before use for testing.

### Polymerase chain reaction

In order to investigate the number of CTG repeats in the 3'-untranslated region (UTR) of the DMPK gene, polymerase chain reaction (PCR) was conducted using the DNA isolated from the target group. The primers used in the polymerase chain reaction can selectively amplify the CTG repeats in the DMPK gene, and the sequences of the primers are as follows:
DM1-F: 5'-GAA GGG TCC TTG TAG CCG GGA A - 3'(SEQ ID NO: 1)
DM1-R: 5'-FAM-GGA GGA TGG AAC ACG GAC GG - 3' (SEQ ID NO: 2)

The primer was labeled with fluorescein (FAM), which is a fluorescent material, so as to check the sizes of amplified products. The polymerase chain reaction was conducted in the conditions of ① 95°C (5 min, once); ② 94°C (1 min) - 60°C (1 min) - 72°C (1 min) (28 cycles); and ③ 60°C (60 min), by mixing 2.5 µL of a forward primer, 2.5 µL of a reverse primer, 1 µL (∼300 ng) of a DNA sample, distilled water, 4 µL of dNTP, 1.5 µL of formamide, and 0.5 µL of Expanded High Fidelity.

The sizes of the amplified products of the DMPK gene, which were obtained by the polymerase chain reaction, were analyzed using a capillary electrophoretic method. 1 µL of the PCR products diluted to 10-fold, 10 µL of formamide, and 0.3 µL of ROX standard were mixed, heated at 95°C for 2 min, immediately cooled on ice, loaded in the ABI 3130xl gene analyzer (Applied Biosystems, USA), and then subjected to capillary electrophoresis. The sizes of amplified products were determined by a comparison with the size of the ROX standard, using the GeneScan Analysis program.

### Multiplex polymerase chain reaction

In order to investigate whether one peak found in the GeneScan Analysis test results was originated from normal homozygote alleles or one normal heterozygote allele of the myotonic dystrophy type 1 patient, semi-quantitative multiplex polymerase chain reaction was conducted.

The multiplex polymerase chain reaction was conducted using the amelogenin gene (GenBank accession No. NM_001143.1) as a standard gene for quantification of the DMPK gene. The amelogenin gene is present at a constant quantity, and may be used as a standard material suitable to relatively compare and quantify the DMPK gene that may have a value of homozygote or heterozygote. Conditions and methods for the semi-quantitative multiplex polymerase chain reaction are in detail described in Korean Patent Registration No. 10-1117798.

Components, concentration thereof, and reaction conditions for the semi-quantitative multiplex polymerase chain reaction were as follows:

### <Primers>

DMPK-GS-F: 5'-FAM-GAAGGGTCCTTGTAGCCGGGAA-3' (SEQ ID NO: 1)
DMPK-GS-R: 5'-GGAGGATGGAACACGGACGG-3' (SEQ ID NO: 2)
Amelogenin-GS-F: 5'-FAM-CCCTGGGCTCTGTAAAGAATAGTG-3' (SEQ ID NO: 3)
Amelogenin-GS-R: 5'-ATCAGAGCTTAAACTGGGAAGCTG-3' (SEQ ID NO: 4)

PCR was conducted in reaction conditions of ① 95°C (5 min, once); ② 94°C (1 min) - 60°C (1 min) - 72°C (1 min) (28 cycles); and ③ 60°C (60 min), using dNTP (200 mM), MgCl₂ (1.25 mM), 2.5 µL of Expanded high fidelity buffer (Roche Diagnostic), 0.75 µL of Expanded high fidelity (Roche Diagnostic), 5% dimethyl sulfoxide (DMSO), primers each 400 nM, and DNA specimen 1 ng.

For gene scanning using PCR amplification products, the ROX standard material was prepared by mixing 900 µL of HiDi formamide (Applied Biosystems, USA) and 30 µL of 500 ROX size standard (Applied Biosystems).

A mixture of 10 µL of the ROX standard material and 1 µL of the obtained amplification products was heated at 95°C for 2 min, immediately cooled on ice, loaded on ABI 3130xl Genetic Analyzer (Applied Biosystems), and then subjected to capillary electrophoresis. The sizes and amounts of amplification products were determined by a comparision with the mixed ROX standard using the GeneScan Analysis program (Applied Biosystems).

It was investigated whether the results obtained from the tested sample are amplification results from a homozygote or a normal heterozygote allele of the patient, by obtaining a relative quantification value of the DMPK gene compared with the amelogenin gene used as a standard gene and then comparing the obtained value with the quantification value of the normal sample that is already known as a homozygote.

### Soutthern blot analysis

A thorough test using southern blot analysis was performed on the individuals who have been determined as being positive as a result of GeneScan Analysis. After the PCR amplification products were separated on the agarose gel, DNA was immobilized on the nitrocellulose filter by allowing the agarose gel to be adsorbed onto the nitrocellulose filter. Then, the filter was hybridized with a probe (SEQ ID NO: 5) labeled with a radioactive isotope (32P), and then the film was exposed to X-ray to investigate bands sensitized with radiation.

As the investigation results, both alleles were determined to include the trinucleotide repeat number within the normal range in cases where one band with a normal size of 9 kb or 10 kb was observed or two bands with sizes of 9 kb and 10 kb were observed. Alternatively, in cases where one normal allele with 9 kb or 10 kb and a band with 10 kb or greater are found, the case was definitely diagnosed as a myotonic dystrophy type 1 patient.

### CTG repeat-primed PCR

A thorough test using CTG repeat-primed PCR was performed on the individuals who have been determined as being positive as a result of GeneScan Analysis.

In order to investigate the number of CTG repeats in the 3'-untranslated region (UTR) of the DMPK gene, CTG repeat-primed PCR was conducted using DNA isolated from the target group. The primers used in the polymerase chain reaction can specifically amplify the CTG repeats in the DMPK gene, and the sequences of the primers are as follows:
DMPK-P1-F: 5'-FAM-GGGGCTCGAAGGGTCCTTGT-3' (SEQ ID NO: 6) 
DMPK-P4CAG-R: 5'-
AGCGGATAACAATTTCACACAGGACAGCAGCAGCAGCAGCAG-3' (SEQ
ID NO: 7)
DMPK-P3-R: 5'-AGCGGATAACAATTTCACACAGGA-3' (SEQ ID NO: 8)

The polymerase chain reaction was conducted in the conditions of ① 95°C (15 min, once); ② 94°C (1 min) - 65 °C (1 min) - 72°C (2 min) (34 cycles); and ③ 72°C (15 min), by mixing 2 µL of the forward primer P1-F, 0.5 uL of the reverse primer P4CAG-R, 1.5 µL of the reverse primer P3-R, 1 uL of DNA sample (∼300 ng), distilled water, 4 µL of dNTP, 1 M betaine, and 0.5 µL of Expanded High Fidelity.

The sizes of the amplified products of the DMPK gene, which were obtained by the polymerase chain reaction, were analyzed using a capillary electrophoretic method. 1 µL of the PCR products diluted to 10-fold, 10 µL of formamide, and 0.3 µL of ROX standard were mixed, heated at 95°C for 2 min, immediately cooled on ice, loaded in the ABI 3130xl gene analyzer Applied Biosystems, USA), and then subjected to capillary electrophoresis. The sizes of amplification products were determined by a comparision with the standard material, using the GeneScan Analysis program.

### Screening of myotonic dystrophy type 1 patients

Myotonic dystrophy type 1 patients were screened using the GeneScan Analysis results and thorough test results, as below.

### <First screening>

① In the GeneScan Analysis results, an individual was determined as being screening-test-negative if the individual is a heterozygote in which two alleles had 34 or less CTG repeats in the 3'-UTR of the DMPK gene and two peaks were observed to validate both two alleles.
② In the GeneScan Analysis results, an individual was determined as being screening-test-positive if the individual had 34 or less CTG repeats in the 3'-UTR of the DMPK gene and one peak was observed. Also, an individual was determined as being screen-test-positive if one peak with 34 or less CTG repeats and one peak with 35 or more CTG repeats were observed, respectively. A thorough test was performed for the screen-test-positive individuals. The thorough test included tests that were performed by southern blot analysis or PCR using CTG repeat-specific primers (CTG-repeat-primed PCR) with respect to DNA isolated from blood, hair, oral mucosal cells, or the like of pregnant women or women of childbearing age.

### <Second screening>

③ In the GeneScan Analysis results, an individual was determined as being thorough-test-negative if the individual was determined as being screening-test-positive since the number of CTG repeats in the 3'-UTR of the DMPK gene was 34 or less and one peak was observed but alleles with 35 or more expanded CTG repeats were not found in the thorough test.
④ In the GeneScan Analysis results, an individual was determined as being thorough-test-positive if the individual was determined as being screening-test-positive since the number of CTG repeats in the 3'-UTR of the DMPK gene was 34 or less and one peak was observed, and alleles with 35 or more expanded CTG repeats were found in the thorough test. Also, an individual was determined as being thorough-test-positive if alleles with 35 or more expanded CTG repeats were found in the screening test and reconfirmed in the thorough test. A prenatal test was performed for the thorough-test-positive pregnant women.

### Example 2: Myotonic dystrophy type 1 screening test for fetuses

### Preparation of DNA sample and diagnostic test method

Pregnant women, determined as being positive in example 1, were diagnosed for myotonic dystrophy type 1, and samples of chorion, amniotic fluid, or cord blood of the pregnant women were collected for diagnosis.

In order to investigate the number of CTG repeats in the 3'-UTR of the DMPK gene, GeneScan Analysis using PCR, southern blot analysis, and CTG repeat-primed PCR were conducted by the same method as in example 1.

### Screening ofynivotonic dystrophy type 1 fetuses

① In the GeneScan Analysis for a fetus, the fetus was determined as being prenatal-test-negative and determined to have a low risk of myotonic dystrophy type 1 if alleles with increased 35 or more CTG repeats in the 3'-UTR of the DMPK gene were not observed.
② In the GeneScan Analysis for a fetus, the fetus was determined as being prenatal-test-positive and determined to have a high risk of myotonic dystrophy type 1 if alleles with increased 35 or more CTG repeats in the 3'-UTR of the DMPK gene were observed.
③ In the GeneScan Analysis for a fetus, the fetus was determined as being a carrier of myotonic dystrophy type 1 and determined to have a low likelihood of the disease if the number of CTG repeats in the 3'-UTR of the DMPK gene was within the range of 35-49.
④ In the GeneScan Analysis for a fetus, the fetus was determined to have a high likelihood of myotonic dystrophy type 1 if the number of CTG repeats in the 3'-UTR of the DMPK gene was 50 or more.
⑤ In the GeneScan Analysis for a fetus, the fetus was determined to have a high likelihood of mild disease if the number of CTG repeats in the 3'-UTR of the DMPK gene was 50-99, a high likelihood of a typical myotonic dystrophy type 1 patient if 100-999, and a high likelihood of a congenital myotonic dystrophy type 1 patient if 1000 or more.

### Example 3: Myotonic dystrophy type 1 screening test for newborn babies

### Preparation of DNA sample and diagnostic test method,

Newborn babies, who was born in or visited Gangnam Severance Hospital, Yonsei University (Seoul, Korea), were diagnosed for myotonic dystrophy type 1, and blood samples were collected from the target group for diagnosis.

In order to investigate the number of CTG repeats in the 3'-UTR of the DMPK gene, GeneScan Analysis using PCR, southern blot analysis, and CTG repeat-primed PCR were conducted by the same method as in example 1.

### Screening of myotonic dystrophy type 1 newborn babies

① In the GeneScan Analysis for a newborn baby, the newborn baby was determined as being screening-test-negative if the individual is a heterozygote in which two alleles have 34 or less CTG repeats in the 3'-UTR of the DMPK gene and two peaks were observed to validate both two alleles.
② In the GeneScan Analysis for a newborn baby, the newborn baby was determined as being screening-test-positive if the individual had 34 or less CTG repeats in the 3'-UTR of the DMPK gene and one peak was observed. Also, the newborn baby was determined as being screen-test-positive if one peak with 34 or less CTG repeats and one peak with 35 or more CTG repeats were observed, respectively. A thorough test was performed for the screen-test-positive individuals. The thorough test included tests that were performed by southern blot analysis or PCR using CTG repeat-specific primer (CTG-repeat-primed PCR) with respect to the nucleic acid (DNS) isolated from blood, oral mucosal cells, hair, or the like of newborn babies.
③ In the GeneScan Analysis for a newborn baby, the newborn baby was determined as being thorough-test-negative if the individual was determined as being screening-test-positive since the number of CTG repeats in the 3'-UTR of the DMPK gene was 34 or less and one peak was observed, but alleles with an expansion of 35 or more CTG repeats were not found in the thorough test.
④ In the GeneScan Analysis for a newborn baby, the newborn baby was determined as being thorough-test-negative if the individual was determined as being screening-test-positive since the number of CTG repeats in the 3'-UTR of the DMPK gene was 34 or less and one peak was observed, but alleles with an expansion of 35 or more CTG repeats were found in the thorough test. Also, the newborn baby was determined as being thorough-test-positive if alleles with an expansion of 35 or more CTG repeats were found in the screening test and reconfirmed in the thorough test.
⑤ In the GeneScan Analysis for a newborn baby, the newborn baby was determined as being a carrier of myotonic dystrophy type 1 and determined to have a low likelihood of the disease if the number of CTG repeats in the 3'-UTR of the DMPK gene was within the range of 35-49.
⑥ In the GeneScan Analysis for a newborn baby, the newborn baby was determined to have a high likelihood of myotonic dystrophy type 1 if the number of CTG repeats in the 3'-UTR of the DMPK gene was 50 or more. Alternatively, the newborn baby was determined to have a high likelihood of mild disease if 50-99, a high likelihood of a typical myotonic dystrophy type 1 patient if 100-999, and a high likelihood of a congenital myotonic dystrophy type 1 patient if 1000 or more.

Although the present invention has been described in detail with reference to the specific features, it will be apparent to those skilled in the art that this description is only for an embodiment and does not limit the scope of the present invention. Thus, the substantial scope of the present invention will be defined by the appended claims and equivalents thereof.

## Claims

1. A diagnostic method of detecting CTG repeats in the dystrophia myotonica-protein kinase (DMPK) gene in order to provide information necessary for the diagnosis of myotonic dystrophy type 1, the method comprising:
(a) performing a first diagnostic test on a target group composed of pregnant women, women of childbearing age, or newborn babies, to screen positive individuals, the first diagnostic test being performed by measuring the number of CTG repeats in the 3'-untranslated region of the DMPK gene in a nucleic acid isolated from a biological sample of the target group, wherein, (i) an individual is determined as being positive if the individual is a homozygote in which two alleles in the DMPK gene have 34 or less CTG repeats or a heterozygote in which two alleles in the DMPK gene have 34 or less and 35 or more CTG repeats, respectively; (ii) an individual is determined as being negative if the individual is a heterozygote in which two alleles in the DMPK gene have 34 or less CTG repeats; or (iii) an individual is determined as being positive if otherwise; and
(b) performing a second diagnostic test on the positive individuals in step (a) to screen positive individuals, the second diagnostic test being performed by measuring the number of CTG repeats in the 3'-untranslated region of the DMPK gene in a nucleic acid isolated from a biological sample of the positive individuals in step (a), wherein, (i) an individual is determined as being negative, if the individual has been determined as being positive as a homozygote in which two alleles in the DMPK gene have 34 or less CTG repeats in the first diagnostic test but the individual has no allele with 35 or more CTG repeats found between the two alleles in the DMPK gene in the second diagnostic test; and (ii) an individual is determined as being positive, if the individual has been the positive individual in the first diagnostic test and the individual has an allele with 35 or more CTG repeats found between the two alleles in the DMPK gene in the second diagnostic test.

2. The method of claim 1, further comprising (c), when the positive individuals in step (b) are pregnant women, performing a third diagnostic test on fetuses to screen positive individuals, the third diagnostic test being performed by measuring the number of CTG repeats in the 3'-untranslated region of the DMPK gene in a nucleic acid isolated from a biological sample of the fetuses, wherein, (i) an individual is determined as being negative if two alleles in the DMPK gene have 34 or less CTG repeats; and (ii) an individual is determined as being positive if an allele with 35 or more CTG repeats is found between the two alleles in the DMPK gene.

3. The method of claim 2, wherein step (c) further comprises determining the positive individual in the third diagnostic test as being a carrier of myotonic dystrophy in cases of 35-49 CTG repeats, a first risk group of myotonic dystrophy in cases of 50-99 CTG repeats, a second risk group of myotonic dystrophy in cases of 100-999 CTG repeats, and a third risk group of myotonic dystrophy in cases of 1000 or more CTG repeats.

4. The method of claim 1, wherein the biological sample is a blood, plasma, serum, urine, cell, hair, or tissue sample.

5. The method of claim 2, wherein the biological sample is chorion, amniotic fluid, placenta, or cord blood.

6. The method of claim 1, wherein the diagnostic method is performed by using hybridization, genetic amplification, immunoassay, or microarray.

7. A computer-implemented identification method for myotonic dystrophy type 1 patients, the method comprising:
(a) performing first screening on a target group composed of pregnant women, women of childbearing age, or newborn babies, using a computer processor, to screen positive individuals, wherein the first screening is performed by (i) screening, as a positive individual, a homozygous individual in which two alleles in the DMPK gene have 34 or less CTG repeats or a heterozygous individual in which two alleles in the DMPK gene have 34 or less and 35 or more CTG repeats, respectively; (ii) screening, as a negative individual, a heterozygous individual in which two alleles in the DMPK gene have 34 or less CTG repeats; or (iii) screening, as a positive individual, if otherwise; and
(b) performing second screening on the positive individuals in step (a), using a computer processor, to screen positive individuals, the second screening is performed by (i) screening, as a negative individual, an individual who has been determined as being a positive individual as a homozygote in which two alleles in the DMPK gene have 34 or less CTG repeats in the first screening but has no allele with 35 or more CTG repeats found between the two alleles in the DMPK gene in the second screening; and (ii) screening, as a positive individual, an individual who has been the positive individual in the first screening and has an allele with 35 or more CTG repeats found between the two alleles in the DMPK gene in the second screening.

8. The method of claim 1, further comprising (c), when the positive individuals screened in step (b) are pregnant women, performing third screening on fetuses, using a computer processor, to screen positive individuals, wherein the third screening is performed by (i) screening, as a negative individual, an individual who has two alleles in the DMPK gene with 34 or less CTG repeats; and (ii) screening, as a positive individual, an individual who has an allele with 35 or more CTG repeats found between the two alleles in the DMPK gene.

9. The method of claim 8, further comprising creating profiles with respect to the likelihood, severity, phenotype, clinical aspects, distribution, disease progression, and related complications of the genetic disease, for the fetuses who are screened as being positive using the computer processor, and creating profiles with respect to the likelihood of the genetic disease, a carrier or not of the genetic disease, and the number of CTG repeats, for the fetuses who are screened as being negative using the computer processor.

10. The method of claim 8, further comprising, screening the positive individual in the third screening as a carrier of myotonic dystrophy in cases of 35-49 CTG repeats, a first risk group of myotonic dystrophy in cases of 50-99 CTG repeats, a second risk group of myotonic dystrophy in cases of 100-999 CTG repeats, and a third risk group of myotonic dystrophy in cases of 1000 or more CTG repeats, using a computer processor.
